# EUROPÄISCHE PATENTSCHRIFT

(11) **EP 4 066 644 B1**
(45) Veröffentlichungstag und Bekanntmachung des Hinweises auf die Patenterteilung: **08.01.2025**
(21) Anmeldenummer: 22162893.6
(22) Anmeldetag: 18.03.2022
(51) Int. Cl.: A01N 41/04, A01N 59/00, A01N 25/02, A01P 1/00, A61L 2/18, A61L 2/22, C02F 1/50, C02F 1/72, C11D 17/00, C11D 3/20, C11D 3/34, C11D 3/36, C11D 3/395, C11D 3/48, C11D 7/02, C11D 7/26, C11D 7/34, C11D 7/36

(54) **DESINFEKTIONSREINIGER, VERFAHREN ZU SEINER HERSTELLUNG UND SEINE VERWENDUNG**
DISINFECTING CLEANER, METHOD FOR ITS PREPARATION AND ITS USE
NETTOYANT DÉSINFECTANT, SON PROCÉDÉ DE PRODUCTION ET SON UTILISATION

(30) Priorität: 19.03.2021 DE 102021202688
(43) Veröffentlichungstag der Anmeldung: 05.10.2022
(73) Patentinhaber: Carela GmbH, 79618 Rheinfelden (DE)
(72) Erfinder: Krumrey, Bernd, 79591 Eimeldingen (DE); Brodbeck, Edith, 79618 Rheinfelden (DE); Reisgys, Michael, 4132 Muttenz (CH)
(74) Vertreter: Friese Goeden Patentanwälte PartGmbB

(56) Entgegenhaltungen:
- EP-A1- 0 656 417
- EP-A2- 0 271 791
- DE-U1- 202021 101 649
- DEMADIS KONSTANTINOS D ET AL: "Chemistry of Organophosphonate Scale Growth lnhibitors: 3. Physicochemical Aspects of 2-Phosphonobutane-1,2,4-Tricarboxylate (PBTC) And Its Effect on CaCO3 Crystal Growth", BIOINORG CHEM APPL, 1 March 2005 (2005-03-01), pages 135 - 149, XP055954485, Retrieved from the Internet <URL:https://www.ncbi.nlm.nih.gov/pmc/articles/PMC2267102/pdf/BCA-03-135.pdf> [retrieved on 20220824], DOI: 10.1155/BCA.2005.135
- PAQUETTE L A ED - PAQUETTE ET AL: "Encyclopaedia of Reagents for Organic Synthesis, POTASSIUM MONOPEROXYSULFATE", ENCYCLOPEDIA OF REAGENTS FOR ORGANIC SYNTHESIS, JOHN WILEY & SONS, LTD, GB, 1 January 1995 (1995-01-01), pages 4265 - 4269, XP002250434, ISBN: 978-0-471-93623-7
- SHIBATA OSAMU ET AL: "New Adsorption Model -Theory, Phenomena and New Concept", JOURNAL OF OLEO SCIENCE, 1 January 2015 (2015-01-01), Japan, pages 1 - 8, XP055954648, Retrieved from the Internet <URL:https://www.researchgate.net/publication/273155349_New_Adsorption_Model_-Theory_Phenomena_and_New_Concept-> [retrieved on 20220824], DOI: 10.5650/jos.ess14213

## Beschreibung

Die Erfindung betrifft einen Desinfektionsreiniger insbesondere für Trinkwasserbehälter, ein Verfahren zu seiner Herstellung sowie seine Verwendung.

Es ist bekannt, dass Oberflächen, die mit Trinkwasser in Berührung kommen, regelmäßig gereinigt und auch desinfiziert werden müssen. Dabei kommen für die Reinigung Reinigungsmittel und für die Desinfektion Desinfektionsmittel zum Einsatz. Da aber zwei sequentielle Verfahrensschritte ausgeführt werden müssen, ist die Reinigung und Desinfektion zeit- und kostenaufwändig. Es besteht somit ein Bedürfnis nach einer Zusammensetzung mit der einfach, schnell und kostengünstig Oberflächen, die mit Trinkwasser in Kontakt kommen, gereinigt und auch desinfiziert werden können.

Die EP 0 656 417 A1 offenbart kalkentfernende Zusammensetzungen, die eine lineare oder verzweigte C1-C6-Alkylsulfonsäure oder Mischungen davon enthalten. Die Alkylsulfonsäuren sind stabil gegenüber Bleichmitteln/Oxidantien.

Ausgehend von diesem Stand der Technik liegt der vorliegenden Erfindung die Aufgabe zugrunde, einen Desinfektionsreiniger sowie ein Verfahren zu seiner Herstellung und seine Verwendung anzugeben, mit denen in einfacher, schneller und kostengünstiger Weise Oberflächen gereinigt und desinfiziert werden können. Weiterhin soll der Desinfektionsreiniger bzw. dessen Rückstände unbedenklich sein, wenn diese mit Trinkwasser in Kontakt kommen.

Die Aufgabe wird erfindungsgemäß durch einen Desinfektionsreiniger gemäß Anspruch 1, ein Verfahren nach Anspruch 13 und eine Verwendung nach Anspruch 14 gelöst. Vorteilhafte Weiterbildungen der Erfindung finden sich in den Unteransprüchen.

Der erfindungsgemäße Desinfektionsreiniger kann zur gleichzeitigen Reinigung und Desinfektion von Oberflächen eingesetzt werden. In einigen Ausführungsformen der Erfindung kann der Desinfektionsreiniger zur gleichzeitigen Reinigung und Desinfektion von Oberflächen eingesetzt werden, welche mit Trinkwasser in Berührung kommen. Reinigung im Sinne der vorliegenden Erfindung bedeutet dabei die Entfernung von Verschmutzungen. Desinfektion im Sinne der vorliegenden Erfindung bedeutet, dass totes oder lebendes Material in einen Zustand versetzt wird, dass es nicht mehr infizieren kann. Der Vorteil der gleichzeitigen Reinigung und Desinfektion ist, dass Zeit und Aufwand gespart werden; es ist kein zweistufiges Verfahren (1. Reinigung und 2. Desinfektion) erforderlich, sondern Reinigung und Desinfektion erfolgen in einem Arbeitsgang.

Der erfindungsgemäße Desinfektionsreiniger enthält neben einer C1-C5-Alkansulfonsäure noch Pentakaliumbis(peroxomonosulfat)-bis(sulfat), welches auch als Caroat bezeichnet wird. Die Zugabe von Caroat weist den Vorteil auf, dass dieser Biozidwirkstoff mit den anderen Rezepturbestandteilen verträglich ist. Er reagiert sauer und ist ebenfalls ein Oxidationsmittel, wie die restliche Mischung. Es fallen keine unlöslichen Stoffe bei der Herstellung und auch bei der Anwendung in einem Trinkwasserbehälter zur Abreinigung der Ablagerungen an. Hierzu genügen bereits 0,05 Gew% bis 0,09 Gew%.

Der erfindungsgemäße Desinfektionsreiniger, insbesondere in den nachstehend beschriebenen Ausführungsformen, weist weitere Vorteile auf. Er ist kein 2-Komponentenprodukt, so dass kein Anmischen beim Anwender erforderlich ist. Ferner ist er flüssig. Damit entfällt das Auflösen eines festen Produktes in Wasser, wodurch bei der Anwendung Zeit gespart werden kann. Der erfindungsgemäße Desinfektionsreiniger löst eisen-, mangan-, und/oder kalkhaltige Ablagerungen auf. Eisen- und kalkhaltige Ablagerungen werden durch den niedrigen, im sauren liegende pH-Wert, manganhaltige Ablagerungen durch die gleichzeitige Anwesenheit von Wasserstoffperoxid als Reduktionsmittel aufgelöst. Potentiell infektiöse Mikroorganismen werden durch den bioziden Wirkstoff Wasserstoffperoxid abgetötet. Biofilme werden dabei durch den als Gasbläschen freiwerdenden Sauerstoff abgehoben und entfernt. Ferner weist der erfindungsgemäße Desinfektionsreiniger Vorteile gegenüber bekannten auf dem Markt erhältlichen salzsauren Produkten auf: das Produkt enthält keine Salzsäure oder Chloride, es ist somit für Edelstahloberflächen geeignet. Eine Lochkorrosion durch Chloride tritt nicht auf. Weiterhin weist der erfindungsgemäße Reiniger gegenüber schwefelsauren und/oder bisulfatsauren Produkten den Vorteil auf, dass durch die Abwesenheit von Sulfaten im erfindungsgemäßen Desinfektionsreiniger keine Umwandlung von Kalkablagerungen in schwer löslichen Gips erfolgt.

In einer Ausführungsform ist die C1-C5-Alkansulfonsäure Methansulfonsäure (CAS 75-75-2).

In einigen Ausführungsformen ist die Phosphonobutantricarbonsäure 2-Phosphonobutan-1,2-4-tricarbonsäure (PBTC, Bayhibit^{®} AM (CAS 037971-36-1)), oder das Salz der Phosphonobutantricarbonsäure ist 2-Phosphonobutan-1,2-4-tricarbonsäurenatrium (Bayhibit^{®} N, (CAS 066669-53-2)).

Der erfindungsgemäße Desinfektionsreiniger enthält Ethylenglycol oder ein sich von Ethylenglycol ableitendes Glycol. In einer Ausführungsform handelt es sich dabei um Triethylenglycol (CAS 112-27-6).

Alle Gew.-% in der vorliegenden Anmeldung beziehen sich auf den erfindungsgemäßen Desinfektionsreiniger. Der Rest auf 100 Gew.-% wird mit Wasser, insbesondere demineralisiertem Wasser, aufgefüllt.

In einigen Ausführungsformen der Erfindung enthält der Desinfektionsreiniger als zusätzlichen Bestandteil weiterhin Phosphorsäure und/oder zumindest eine organische Säure. Die zumindest eine organische Säure kann in einigen Ausführungsformen der Erfindung ausgewählt sein aus Oxalsäure und/oder Citronensäure und/oder Ascorbinsäure und/oder Äpfelsäure und/oder Weinsäure. Für die Zwecke der vorliegenden Beschreibung wird auch ein Phosphorsäuremonoalkylester, insbesondere Methylester, als organische Säure bezeichnet.

In dem erfindungsgemäßen Desinfektionsreinigers liegt die Alkansulfonsäure in einer Menge von 10 Gew.-% bis 50 Gew.-% vor. In einer Ausführungsform des erfindungsgemäßen Desinfektionsreinigers liegt die Alkansulfonsäure in einer Menge von 20 Gew.-% bis 40 Gew.-% vor. In wiederum einer anderen Ausführungsform des erfindungsgemäßen Desinfektionsreinigers liegt die Alkansulfonsäure in einer Menge von 35 Gew.-% vor.

In einer Ausführungsform des erfindungsgemäßen Desinfektionsreinigers liegt die Phosphorsäure und/oder die organische Säure in einer Menge von 1 Gew.-% bis 20 Gew.-% vor. In einer anderen Ausführungsform des erfindungsgemäßen Desinfektionsreinigers liegt die Phosphorsäure und/oder die organische Säure in einer Menge von 2 Gew.-% bis 10 Gew.-% vor. In wiederum einer anderen Ausführungsform des erfindungsgemäßen Desinfektionsreinigers liegt die Phosphorsäure und/oder die organische Säure in einer Menge von 3 Gew.-% vor.

Der erfindungsgemäße Desinfektionsreiniger enthält Phosphonobutantricarbonsäure oder das Salz der Phosphonobutantricarbonsäure in einer Menge von 0,1 Gew.-% bis 10 Gew.-%. In einer Ausführungsform des erfindungsgemäßen Desinfektionsreinigers enthält dieser Phosphonobutantricarbonsäure oder das Salz der Phosphonobutantricarbonsäure in einer Menge von 0,2 Gew.-% bis 5 Gew.-%. In wiederum einer anderen Ausführungsform des erfindungsgemäßen Desinfektionsreinigers enthält dieser Phosphonobutantricarbonsäure oder das Salz der Phosphonobutantricarbonsäure in einer Menge von 0,5 Gew.-%.

Der erfindungsgemäße Desinfektionsreiniger enthält Ethylenglycol oder ein sich von Ethylenglycol ableitendes Glycol in einer Menge von 0,1 Gew.-% bis 5 Gew.-%. In einer Ausführungsform des erfindungsgemäßen Desinfektionsreinigers enthält dieser Ethylenglycol oder ein sich von Ethylenglycol ableitendes Glycol in einer Menge von 0,2 Gew.-% bis 3 Gew.-%. In wiederum einer anderen Ausführungsform des erfindungsgemäßen Desinfektionsreinigers enthält dieser Ethylenglycol oder ein sich von Ethylenglycol ableitendes Glycol in einer Menge von 0,5 Gew.-%.

Der erfindungsgemäße Desinfektionsreiniger enthält Wasserstoffperoxid in einer Menge von 1 Gew.-% bis 30 Gew.-%. In einer Ausführungsform des erfindungsgemäßen Desinfektionsreinigers enthält dieser Wasserstoffperoxid in einer Menge von 5 Gew.-% bis 20 Gew.-%. In wiederum einer anderen Ausführungsform des erfindungsgemäßen Desinfektionsreinigers enthält dieser Wasserstoffperoxid in einer Menge von 8 Gew.-%.

In einigen Ausführungsformen kann der Desinfektionsreiniger mindestens eine weitere Verbindung enthalten, welche ausgewählt ist aus der Gruppe nichtionischer Tenside und/oder kationischer Tenside und/oder anionischer Tenside und/oder einem weiteren Komplexbildner und/oder Lösevermittler und/oder zumindest ein weiteres Biozid, d.h. ein anderes Biozid als Wasserstoffperoxid. In einigen Ausführungsformen kann jede der weiteren Verbindungen in einer Menge von 0,1 Gew.-% bis 10 Gew.-% vorhanden sein. In anderen Ausführungsformen kann jede der weiteren Verbindungen in einer Menge von 2 Gew.-% bis 8 Gew.-% vorhanden sein. In wiederum anderen Ausführungsformen kann jede der weiteren Verbindungen in einer Menge von 5 Gew.-% vorliegen.

In einigen Ausführungsformen kann der Desinfektionsreiniger als nichtionisches Tensid zumindest einem Alkohol mit C10-C16 enthalten, welcher ethoxyliert oder propoxyliert sein kann (CAS 69227-22-1). In einigen Ausführungsformen kann das nichtionische Tensid in einer Menge von 0,1 Gew.-% bis 10 Gew.% vorliegen.

In einigen Ausführungsformen kann der Desinfektionsreiniger als kationisches Tensid zumindest eine quaternäre Ammoniumverbindung sein. In einigen Ausführungsformen kann der Desinfektionsreiniger als anionisches Tensid zumindest ein längerkettiges Alkylsulfonat und/oder zumindest eine längerkettige Alkylsulfonsäure enthalten. Unter einem längerkettigen Alkylsulfonat bzw. einer längerkettigen Alkylsulfonsäure wird für die Zwecke der vorliegenden Erfindung ein Alkylsulfonat bzw. eine Alkylsulfonsäure verstanden, welche eine größeren Kettenlänge als die im Desinfektionsreiniger ohnehin vorhandene C1-C5-Alkansulfonsäure aufweist. In einigen Ausführungsformen kann das längerkettige Alkylsulfonat bzw. die längerkettige Alkylsulfonsäure eine Kettenlänge von etwa C8 bis etwa C20 oder von etwa C8 bis etwa C18 aufweisen.

In einigen Ausführungsformen kann der Desinfektionsreiniger als weiteren Komplexbildner zumindest ein Phosphonat enthalten. Das Phosphonat kann ausgewählt sein unter 1-Hydroxyethan-(1,1-diphosphonsäure) und/oder 3-Carboxy-3-phosphonoadipinsäure. In anderen Ausführungsformen kann der Komplexbildner alternativ oder zusätzlich Nitrilotriessigsäure und/oder Dicarbonsäure und/oder Polycarbonsäure enthalten.

In einigen Ausführungsformen kann der Desinfektionsreiniger als Lösevermittler Natriumcumolsulfonat (CAS 15763-76-5) enthalten. Der Lösevermittler kann in einigen Ausführungsformen in einer Menge von etwa 0,1 Gew-% bis etwa 10 Gew.-% vorliegen.

In einigen Ausführungsformen kann der Desinfektionsreiniger als weiteren Biozidwirkstoff Quats enthalten.

Erfindungsgemäß wird weiterhin ein Verfahren zur Herstellung des erfindungsgemäßen Desinfektionsreinigers angegeben, bei welchem die vorstehend angegebenen Bestandteile in Wasser gemischt werden.

Der erfindungsgemäße Desinfektionsreiniger kann zur Reinigung und gleichzeitigen Desinfektion von Oberflächen verwendet werden, welche mit Wasser in Kontakt kommen. Insbesondere kann der erfindungsgemäße Desinfektionsreiniger dazu eingesetzt werden, Trinkwasser-führende Oberflächen zu reinigen und zu desinfizieren, beispielsweise die Innenflächen von Filteranlagen, Rohrleitungen, Pumpen, Armaturen Wärmetauschern, Kühltürmen oder Behältern. Solche Anlagen können Teil einer Trinkwasserversorgung sein. In anderen Ausführungsformen können solche Anlagen in der Getränke- und Lebensmittelindustrie eingesetzt werden.

Der Desinfektionsreiniger ist als anwendungsbereite Lösung oder als mit Wasser zu verdünnendes Konzentrat herstellbar. Er kann mit einem Niederdrucksprühgerät auf die Oberflächen aufgetragen werden. Nach der Einwirkung kann er mit Wasser abgespült werden. Rohrleitungen, Installationen, Armaturen usw. können mit dem Desinfektionsreiniger befüllt oder gespült werden.

Weitere beispielhafte Anwendungen des Desinfektionsreinigers sind die Entkalkung von Haushaltsgeräten (Kaffeemaschinen, Boiler, Waschmaschinen und andere). Der Desinfektionsreiniger wirkt dabei schneller als Citronensäure- oder Sulfaminsäure-haltige Produkte. Weiterhin kann der Desinfektionsreiniger beispielsweise als Sanitärreiniger für beispielsweise Porzellan, Edelstahl oder verchromte Oberflächen eingesetzt werden. Schließlich kann der Desinfektionsreiniger in Schwimmbädern eingesetzt werden, beispielsweise für die Reinigung und Desinfektion sowie Entfernung von Ablagerungen. Solche Ablagerungen können sein Kalk, Schmutzränder usw. an wasserberührten Oberflächen, wie Fliesen, Kacheln, Kunststoffe, Ablaufrosten, Böden, Becken, Solebecken, Thermalbecken, Beckenrändern, sowie deren Einbauten, beispielsweise Leitern oder Griffe.

### Beispiel

Das folgende Beispiel soll die Erfindung näher erläutern:
Es wurde ein Desinfektionsreiniger mit der folgenden Zusammensetzung hergestellt (die Mengenangaben sind in Gew.-% angegeben, bezogen auf den fertigen Desinfektionsreiniger):
Methansulfonsäure (CAS 75-75-2) 35%; Pentakaliumbis(peroxomonosulfat)-bis(sulfat) 0,09%; Phosphorsäure (CAS 7664-38-2) 3%; 2-Phosphonobutan-1,2,4-tricarbonsäure, (PBTC, Bayhibit^{®} AM (CAS 037971-36-1)) 0,5%, Triethylenglycol (CAS 112-27-6) 0,5%; Wasserstoffperoxid (CAS 7722-84-1) 8%, und demineralisiertes Wasser zu 100%.

Mit diesem Desinfektionsreiniger wurde die Oberfläche eines Trinkwasserbehälters behandelt. Es wurde festgestellt, dass sowohl eine Desinfektion als auch eine Reinigung erfolgte. Es fielen keine unlöslichen Stoffe bei der Herstellung und auch bei der Anwendung in einem Trinkwasserbehälter zur Abreinigung der Ablagerungen an, so dass Rückstände leicht ausgespült werden konnten.

Selbstverständlich ist die Erfindung nicht auf die dargestellten Ausführungsformen beschränkt. Die vorstehende Beschreibung ist daher nicht als beschränkend, sondern als erläuternd anzusehen. Die nachfolgenden Ansprüche sind so zu verstehen, dass ein genanntes Merkmal in zumindest einer Ausführungsform der Erfindung vorhanden ist. Dies schließt die Anwesenheit weiterer Merkmale nicht aus. Sofern die Ansprüche und die vorstehende Beschreibung "erste" und "zweite" Ausführungsformen definieren, so dient diese Bezeichnung der Unterscheidung zweier gleichartiger Ausführungsformen, ohne eine Rangfolge festzulegen.

## Patentansprüche

1. Desinfektionsreiniger, enthaltend:
(a) 10 Gew.-% bis 50 Gew.-% zumindestens einer C1-C5-Alkansulfonsäure,
(b) 0,05 Gew.-% bis 0,09 Gew.-% Pentakalium-bis(peroxomonosulfat)-bis(sulfat)
(c) 0,1 Gew.-% bis 10 Gew.-% einer Phosphonobutantricarbonsäure oder eines Salzes der Phosphonobutantricarbonsäure
(d) 0,1 Gew.-% bis 5 Gew.-% Ethylenglycol oder eines sich von Ethylenglykol ableitenden Glycols
(e) 1 Gew.-% bis 30 Gew.-% Wasserstoffperoxid und
(f) Wasser.

2. Desinfektionsreiniger nach Anspruch 1, **dadurch gekennzeichnet, dass** dieser zusätzlich Phosphorsäure und/oder eine organische Säure enthält.

3. Desinfektionsreiniger nach Anspruch 1 oder 2, **dadurch gekennzeichnet, dass** die C1-C5-Alkansulfonsäure Methansulfonsäure enthält oder daraus besteht.

4. Desinfektionsreiniger nach Anspruch 1, **dadurch gekennzeichnet, dass** die mindestens eine Phosphonobutantricarbonsäure 2-Phosphonobutan-1,2-4-tricarbonsäure oder das Salz der Phosphonobutantricarbonsäure 2-Phosphonobutan-1,2-4-tricarbonsäurenatrium enthält oder daraus besteht.

5. Desinfektionsreiniger nach einem der Ansprüche 1 bis 4, **dadurch gekennzeichnet, dass** das von Ethylenglycol abgeleitete Glycol Triethylenglycol enthält oder daraus besteht.

6. Desinfektionsreiniger nach einem der Ansprüche 1 bis 5 **dadurch gekennzeichnet, dass** die Alkylsulfonsäure in einer Menge von 20 Gew.-% bis 40 Gew.-% oder etwa 35 Gew.-% vorliegt.

7. Desinfektionsreiniger nach einem der Ansprüche 2 bis 6, **dadurch gekennzeichnet, dass** die Phosphorsäure und/oder die organische Säure in einer Menge von 1 Gew.-% bis 20 Gew.-% oder 2 Gew.-% bis 10 Gew.-% oder etwa 3 Gew.-% vorliegt.

8. Desinfektionsreiniger nach einem der Ansprüche 1 bis 7, **dadurch gekennzeichnet, dass** die Phosphonobutantricarbonsäure oder das Salz der Phosphonobutantricarbonsäure in einer Menge von 0,2 Gew.-% bis 5 Gew.-% oder etwa 0,5 Gew.-% vorliegt.

9. Desinfektionsreiniger nach einem der Ansprüche 1 bis 8, **dadurch gekennzeichnet, dass** das Ethylenglycol oder das sich von Ethylenglykol ableitende Glycol in einer Menge von 0,2 Gew.-% bis 3 Gew.-% oder etwa 0,5 Gew.-% vorliegt.

10. Desinfektionsreiniger nach einem der Ansprüche 1 bis 9, **dadurch gekennzeichnet, dass** das Wasserstoffperoxid in einer Menge von 5 Gew.-% bis 20 Gew.-% oder etwa 8 Gew.-% vorliegt.

11. Desinfektionsreiniger nach einem der Ansprüche 1 bis 10, **dadurch gekennzeichnet, dass** dieser mindestens eine weitere Verbindung enthält, ausgewählt aus zumindest einem nichtionischen Tensid und/oder zumindest einem kationischen Tensid und/oder zumindest einem anionischen Tensid und/oder zumindest einem weiteren Komplexbildner und/oder zumindest einem Lösevermittler und/oder zumindest einem weiteren Biozid.

12. Desinfektionsreiniger nach Anspruch 11, **dadurch gekennzeichnet, dass** jede der weiteren Verbindungen in einer Menge von 0,05 Gew.-% bis 0,1 Gew.-% oder 0,1 Gew.-% bis 10 Gew.-% oder 2 Gew.-% bis 8 Gew.-% oder etwa 5 Gew.-% vorliegt.

13. Verfahren zur Herstellung des Desinfektionsreinigers nach einem der Ansprüche 1 bis 12, bei welchem dessen Bestandteile eingewogen und in Wasser gemischt werden.

14. Nicht-therapeutische Verwendung des Desinfektionsreinigers nach einem der Ansprüche 1 bis 12 zur Reinigung und gleichzeitigen Desinfektion von Oberflächen, welche mit Wasser in Kontakt kommen.

## Claims

1. Disinfectant cleaner, comprising:
(a) 10 wt.% to 50 wt.% of at least one C1-C5 alkanesulfonic acid,
(b) 0.05 wt.% to 0.09 wt.% of pentapotassium bis(peroxymonosulfate) bis(sulfate)
(c) 0.1 wt.% to 10 wt.% of a phosphonobutane tricarboxylic acid or a salt of the phosphonobutane tricarboxylic acid
(d) 0.1 wt.% to 5 wt.% of ethylene glycol or a glycol derived from ethylene glycol
(e) 1 wt.% to 30 wt.% of hydrogen peroxide and
(f) water.

2. Disinfectant cleaner according to claim 1, **characterized in that** it additionally contains phosphoric acid and/or an organic acid.

3. Disinfectant cleaner according to claim 1 or 2, **characterized in that** the C1-C5 alkanesulfonic acid contains or consists of methanesulfonic acid.

4. Disinfectant cleaner according to claim 1, **characterized in that** the at least one phosphonobutane tricarboxylic acid contains or consists of 2-phosphonobutane-1,2,4-tricarboxylic acid or the salt of the phosphonobutane tricarboxylic acid contains or consists of 2-phosphonobutane-1,2,4-tricarboxylic acid sodium.

5. Disinfectant cleaner according to any one of claims 1 to 4, **characterized in that** the glycol derived from ethylene glycol contains or consists of triethylene glycol.

6. Disinfectant cleaner according to any one of claims 1 to 5, **characterized in that** the alkylsulfonic acid is present in an amount of 20 wt.% to 40 wt.% or about 35 wt.%.

7. Disinfectant cleaner according to any one of claims 2 to 6, **characterized in that** the phosphoric acid and/or the organic acid is present in an amount of 1 wt.% to 20 wt.% or of 2 wt.% to 10 wt.% or about 3wt.%.

8. Disinfectant cleaner according to any one of claims 1 to 7, **characterized in that** the phosphonobutane tricarboxylic acid or the salt of the phosphonobutane tricarboxylic acid is present in an amount of 0.2 wt.% to 5 wt.% or about 0.5 wt.%.

9. Disinfectant cleaner according to any one of claims 1 to 8, **characterized in that** the ethylene glycol or the glycol derived from ethylene glycol is present in an amount of 0.2 wt.% to 3 wt.% or about 0.5 wt.%.

10. Disinfectant cleaner according to any one of claims 1 to 9, **characterized in that** the hydrogen peroxide is present in an amount of 5 wt.% to 20 wt.% or about 8 wt.%.

11. Disinfectant cleaner according to any one of claims 1 to 10, **characterized in that** it contains at least one further compound selected from at least one non-ionic surfactant and/or at least one cationic surfactant and/or at least one anionic surfactant and/or at least one further complexing agent and/or at least one solubilizer and/or at least one further biocide.

12. Disinfectant cleaner according to claim 11, **characterized in that** each of the further compounds is present in an amount of 0.05 wt.% to 0.1 wt.% or of 0.1 wt.% to 10 wt.% or of 2 wt.% to 8 wt.% or about 5 wt.%.

13. Method for producing the disinfectant cleaner according to any one of claims 1 to 12, in which its ingredients are weighed in and mixed in water.

14. Non-therapeutic use of the disinfectant cleaner according to any one of the claims 1 to 12 for cleaning and simultaneously disinfecting surfaces that come into contact with water.

## Revendications

1. Nettoyant désinfectant, contenant :
(a) 10 % en poids à 50 % en poids d'au moins un acide alcanesulfonique en C1-C5,
(b) 0,05 % en poids à 0,09 % en poids de bis(peroxomono)-bis(sulfate) de pentacalium
(c) 0,1 % en poids à 10 % en poids d'un acide phosphonobutane tricarboxylique ou d'un sel de l'acide phosphonobutane tricarboxylique
(d) 0,1 % en poids à 5 % en poids d'éthylène glycol ou d'un glycol dérivé de l'éthylène glycol
(e) 1 % en poids à 30 % en poids de peroxyde d'hydrogène, et
(f) de l'eau.

2. Nettoyant désinfectant selon la revendication 1,
**caractérisé en ce qu'**il contient en supplément de l'acide phosphorique et/ou un acide organique.

3. Nettoyant désinfectant selon la revendication 1 ou 2,
**caractérisé en ce que** l'acide alcanesulfonique en C1-C5 contient ou consiste en acide méthanesulfonique.

4. Nettoyant désinfectant selon la revendication 1,
**caractérisé en ce que** ledit au moins un acide phosphonobutane tricarboxylique contient ou consiste en acide 2-phosphonobutane-1,2-4-tricarboxylique, ou le sel de l'acide phosphonobutane tricarboxylique contient ou consiste en acide 2-phosphonobutane-1,2-4-tricarboxylique sodique.

5. Nettoyant désinfectant selon l'une des revendications 1 à 4,
**caractérisé en ce que** le glycol dérivé de l'éthylène glycol contient ou consiste en triéthylène glycol.

6. Nettoyant désinfectant selon l'une des revendications 1 à 5,
**caractérisé en ce que** l'acide alkylsulfonique est présent en une quantité de 20 % en poids à 40 % en poids ou d'environ 35 % en poids.

7. Nettoyant désinfectant selon l'une des revendications 2 à 6,
**caractérisé en ce que** l'acide phosphorique et/ou l'acide organique est présent en une quantité de 1 % en poids à 20 % en poids ou de 2 % en poids à 10 % en poids ou d'environ 3 % en poids.

8. Nettoyant désinfectant selon l'une des revendications 1 à 7,
**caractérisé en ce que** l'acide phosphonobutane tricarboxylique ou le sel de l'acide phosphonobutane tricarboxylique est présent en une quantité de 0,2 % en poids à 5 % en poids ou d'environ 0,5 % en poids.

9. Nettoyant désinfectant selon l'une des revendications 1 à 8,
**caractérisé en ce que** l'éthylène glycol ou le glycol dérivé de l'éthylène glycol est présent en une quantité de 0,2 % en poids à 3 % en poids ou d'environ 0,5 % en poids.

10. Nettoyant désinfectant selon l'une des revendications 1 à 9, **caractérisé en ce que** le peroxyde d'hydrogène est présent en une quantité de 5 % en poids à 20 % en poids ou d'environ 8 % en poids.

11. Nettoyant désinfectant selon l'une des revendications 1 à 10, **caractérisé en ce qu'**il contient au moins un autre composé choisi parmi au moins un agent tensioactif non ionique et/ou au moins un agent tensioactif cationique et/ou au moins un agent tensioactif anionique et/ou au moins un autre agent complexant et/ou au moins un agent solubilisant et/ou au moins un autre biocide.

12. Nettoyant désinfectant selon la revendication 11,
**caractérisé en ce que** chacun des autres composés est présent en une quantité de 0,05 % en poids à 0,1 % en poids ou de 0,1 % en poids à 10 % en poids ou de 2 % en poids à 8 % en poids ou d'environ 5 % en poids.

13. Procédé de préparation du nettoyant désinfectant selon l'une des revendications 1 à 12, dans lequel ses composants sont pesés et mélangés dans de l'eau.

14. Utilisation non thérapeutique du nettoyant désinfectant selon l'une des revendications 1 à 12 pour le nettoyage et la désinfection simultanée de surfaces qui viennent en contact avec l'eau.
